Europäisches Patentamt

(19) European Patent Office

Office européen des brevets

(11) EP 0 962 139 A1

(12) EUROPEAN PATENT APPLICATION

(43) Date of publication:
08.12.1999 Bulletin 1999/49

(51) Int. Cl.⁶: **A01N 53/00**, A01N 25/18,
A01M 1/20, A61L 9/12
// (A01N53/00, 25:18)

(21) Application number: 99110212.0

(22) Date of filing: 26.05.1999

(84) Designated Contracting States:
AT BE CH CY DE DK ES FI FR GB GR IE IT LI LU
MC NL PT SE
Designated Extension States:
AL LT LV MK RO SI

(30) Priority: 28.05.1998 JP 14742098
12.11.1998 JP 32204698
29.01.1999 JP 2235199

(71) Applicant:
**Sumitomo Chemical Company, Limited**
**Osaka-shi, Osaka-fu (JP)**

(72) Inventor: **Ishiwatari, Takao**
**Toyonaka-shi, Osaka-fu (JP)**

(74) Representative:
**VOSSIUS & PARTNER**
**Siebertstrasse 4**
**81675 München (DE)**

(54) **Method and apparatus for pest control**

(57) Stable and lasting excellent effects on the control of pests can be attained without using any heat source by a pest control method which includes raising a relative air current on the surface of a pest control material carrying on a support at least one active agent selected from the group consisting of 1-ethynyl-2-methyl-2-pentenyl 3-(2-chloro-2-fluorovinyl)-2,2-dimethylcyclopropanecarboxylate, 2,3,5,6-tetrafluoro-4-methylbenzyl 3-(2-methyl-1-propenyl)-2,2-dimethylcyclopropanecarboxylate, 2,3,5,6-tetrafluoro-4-methylbenzyl 3-(2-chloro-2-fluorovinyl)-2,2-dimethylcyclopropanecarboxylate, and 2,3,5,6-tetrafluoro-4-methylbenzyl 3-(1-propenyl)-2,2-di methylcyclopropanecarboxylate, to vaporize and diffuse the active agent in the air.

Fig.3

## Description

The present invention relates to a pest control method, and more particularly, it relates to a method for the extermination of pests. Also, the present invention relates to a pest control apparatus.

For killing insects such as mosquitoes or preventing their bites, there have been used so far mosquito coils or electric anti-mosquito devices of the tablet or liquid type. Active agents are vaporized by making use of heat generated from the combustion of the mosquito coil in the former case or by heat generation up to 120-170°C on a heating base connected to an electric source in the latter case.

The above means, however, may involve a danger such as occurrence of fires or formation of burns, and the use of a heat source such as fire may impose limitations on their application in the open air or under unattended conditions. In addition, since active agents are heated at high temperatures, it is hard to prevent their thermal decomposition, even if inhibitors or other additives are used.

WO 96/04786 discloses a pest control method using no heat source, in which active agents such as empenthrin are vaporized and diffused by bringing them into contact with an air current raised by the use of a blowing means. In some cases, however, satisfactory effects cannot be attained.

It is the object of the present invention to provide an improved pest control method and apparatus. This object is achieved with the subject-matter according to the respective claims.

The applicant has found that specific active agents can be vaporized and diffused in the air with high efficiency by raising a relative air current on the surface of a pest control material carrying the active agents on a support, so that excellent effects on the control of pests can be attained, thereby completing the present invention.

Thus, the present invention provides a pest control method which comprises raising a relative air current on the surface of a pest control material carrying on a support at least one active agent selected from the group consisting of 1-ethynyl-2-methyl-2-pentenyl 3-(2-chloro-2-fluorovinyl)-2,2-dimethylcyclopropanecarboxylate, 2,3,5,6-tetrafluoro-4-methylbenzyl 3-(2-methyl-1-propenyl)-2,2-dimethylcyclopropanecarboxylate, 2,3,5,6-tetrafluoro-4-methylbenzyl 3-(2-chloro-2-fluorovinyl)-2,2-dimethylcyclopropanecarboxylate, and 2,3,5,6-tetrafluoro-4-methylbenzyl 3-(1-propenyl)-2,2-di methylcyclopropanecarboxylate, to vaporize and diffuse the active agent in the air.

Another pest control method of the present invention includes the steps of providing a carrier having a pest control agent capable of vaporizing by the contact with air, and moving the carrier and thereby generating a stream of air relative to the carrier.

A pest control apparatus of the present invention comprises a carrier having a pest control agent capable of vaporizing by the contact with air, and means for moving the carrier and thereby generating a stream of air relative to the carrier.

In another aspect of the present invention, a pest control apparatus comprises a carrier and a motor. The carrier, which has a pest control agent capable of vaporizing by the contact with air, includes a shaft and a portion securely supported by the shaft and extending substantially outwardly and radially from the shaft. The motor rotates the carrier about the shaft, which generates a stream of air relative to the carrier to help the pest control agent vaporize.

In another aspect of the present invention, a pest control apparatus comprises a carrier including a pest control agent capable of vaporizing by the contact with air. The carrier has a shaft and a blade. The blade is securely supported by the shaft and extended substantially outwardly and radially from the shaft. In particular, the blade is inclined to a plane perpendicular to the shaft. A bearing is provided for supporting the carrier so that the carrier can rotate about the shaft. The apparatus further comprises a motor and a fan. The fan is drivingly connected with the motor so that the fan rotates to provide said inclined blade of the carrier with a stream of air which causes said carrier to rotate. This generates another stream of air relative to the carrier to help the pest control agent vaporize.

The carrier may include a plurality of blades equally positioned about the shaft, so that the carrier is in the form of propeller.

Also, the apparatus may comprise a breathable member surrounding the carrier.

In another aspect of the present invention, a pest control apparatus comprises a carrier which includes a pest control agent capable of vaporizing by the contact with air. The carrier has a bearing portion, a horizontal portion extending substantially outwardly and radially from the bearing portion, and a vertical portion extending substantially downwardly from the horizontal portion. A shaft is drivingly connected with a motor for rotation and is engaged with the first bearing so that the carrier can rotate about the shaft by a frictional contact between the bearing portion and the shaft generated at the rotation of said shaft. This forms a stream of air relative to the carrier to help the pest control agent vaporize. A fan is securely supported by the shaft so that the fan rotates together with the shaft to provide the carrier with an additional stream of air which accelerates a vaporization of the carrier. Preferably, the fan is a centrifugal fan and positioned inside the carrier.

In another aspect of the present invention, a pest control apparatus comprises a rotatable shaft, an arm securely supported by the shaft and extending outwardly and radially from the shaft, a carrier supported by the arm and including a pest control agent capable of vaporizing by the contact with air, and a motor for rotating the shaft, which gives rise to a stream of air relative to the carrier to help the pest control agent vaporize. The shaft securely supports a blade inclined to a plane perpendicular to the shaft, which gives rise to an additional stream of air to accelerates a vaporization of the carrier.

In another aspect of the preset invention, a pest control apparatus comprises a vertical member having first and second ends. The vertical member is fixed at the first end. A horizontal member is connected to the second end of the vertical member so that the horizontal member can rotate about the second end of the vertical member. Also, the horizontal member has first and second arms extending opposite directions from the second end of the vertical member. A carrier, having a pest control agent capable of vaporizing by the contact with air, is supported by the first arm of the horizontal member. On the other hand, a counterweight is supported by the second arm of the horizontal member so that it is balanced against the carrier. The apparatus further includes means for rotating the horizontal member about the second end of the vertical member and thereby generating a stream of air relative to the carrier. Preferably, means for rotating the horizontal member includes a motor mounted in the carrier and a propeller drivingly connected with the motor. Also, the counterweight includes a battery for providing electricity to the motor, and an electric circuit connects between the motor and the battery.

With the arrangement, the pest control agent in the carrier is effectively vaporized and then diffused by the stream of air generated by the movement of the carrier.

In the following preferred embodiments of the present inventions are exemplified by means of the drawings.

Fig. 1 is a top plan view of a pest control material used in the insecticidal test of Example 1;
Fig. 2 is a perspective view of the same pest control material as shown in Fig. 1;
Fig. 3 is an exploded perspective view of a pest control device of the first embodiment according to the present invention;
Fig. 4 is a perspective view of a pest control device of the second embodiment according to the present invention;
Fig. 5 is an exploded perspective view of a centrifugal fan and a carrier incorporated in the pest control device in Fig. 4;
Fig. 6 is a perspective view of a pest control device of the third embodiment according to the present invention;
Fig. 7 is a perspective view of a pest control agent support incorporated in the pest control device in Fig. 6; and
Fig. 8 is a perspective view of a pest control device of the fourth embodiment according to the present invention.

The active agents used in the method of the present invention can be prepared by esterification of alcohols and carboxylic acids both well known in the art according to the ordinary procedures.

For these active agents, there may exist optical isomers based on the presence of an asymmetric carbon atom, and the optical isomers having biological activity, whether they have either R- or S-configuration, and their mixtures at any ratio are, of course, included within the scope of the present invention. Furthermore, there may exist geometrical isomers based on the presence of a cyclopropane ring and/or a double bond, and the geometrical isomers having biological activity, whether they are either in Z- or E-form, and their mixtures at any ratio are, of course, included within the scope of the present invention.

Among the above active agents, preferably used is 2,3,5,6-tetrafluoro-4-methylbenzyl 3-(2-chloro-2-fluorovinyl)-2,2-dimethylcyclopropanecarboxylate.

The pest control material used in the method of the present invention is obtained, for example, by dissolving an active agent in an organic solvent, if necessary, and then impregnating into or coating on a support, or by kneading with a molten support, followed by molding.

The support used in the method of the present invention may be those which can carry an active agent and can vaporize the active agent to a proper extent by a relative air current raised on the surface thereof. The support may include solid supports, those which have been made into a gel form by their impregnation with water, and those which can be melted for kneading to incorporate an active agent.

Specific examples of the support are paper, nonwoven fabric, cloth, spongy plastics, resin films, polyurethane, sponge, dispersible silica, organic montmorillonite, sodium stearate, and carageenan. From the viewpoints of high vaporization efficiency and easy preparation, sheet-shaped supports made of paper, nonwoven fabric, cloth, spongy plastics or resin films are preferably used. If required, the sheet-shaped supports can also be folded in a suitable form, laminated in part, formed into a honeycomb structure, or provided with concave and convex portions on their surfaces, to enlarge the surface area relative to the volume of the supports.

The amount of active agent carried on the support in the pest control material, although it may vary with the purpose, scene and period of application, as well as other factors, is usually about 0.01 to 100 g, preferably about 0.1 to 100 g, and more preferably about 0.1 to 10 g.

The active agent carried on the support in the pest control material is vaporized and diffused into the air by raising a relative air current on the surface of the pest control material, and the effects on the control of pests can be exhibited by such a vaporized and diffused active agent.

The term "relative air current" as used herein refers to an air current raised by relative movement between the pest control material and the air. More particularly, when a pest control material remains fixed, an relative air current may be raised on the surface of the pest control material by allowing a wind to blow the pest control material by the use of a blowing means. The relative air current may also be raised by allowing the pest control material itself to move in the air. These techniques may be, of course, combined.

The blowing means may typically utilize a blower such as a small-sized electric fan.

The movement of a pest control material itself can be achieved, for example, by spinning a windmill- or propeller-shaped pest control material carrying an active ingredient, or by revolving a mobile carrying an active ingredient, or by rotating a slit rotator carrying an active ingredient.

In the method of the present invention, the velocity of a relative air current on the surface of a pest control material should be enough to vaporize and diffuse an active agent carried on a support in the pest control material in an amount exhibiting satisfactory effects on the control of pests, and it is usually in the range of about 0.1 to 10 m/sec.

The pest control material is disposed so as to allow a wind to pass therethrough, for example, disposed so that the surface of a pest control material comes parallel to the direction of a wind blowing from a blowing means. When the method of the present invention is put into practice, a pest control material can be disposed, for example, in a cylindrical tube having both open ends and a wind can be allowed to blow the pest control material by the use of a blowing means provided at one of the open ends of the cylindrical tube. The cylindrical tube may have a circular or polygonal cross section, such as trigonal tetragonal or hexagonal, and may be made of various materials including resins and cardboards. Specific examples of the resin are synthetic resins such as polyethylene; polypropylene; copolymers of ethylene with polar group-containing monomers, e.g., ethylene-vinyl acetate copolymers, ethylene-methyl (meth)acrylate copolymers, ethylene-ethyl acrylate copolymers, ethylene-vinyl acetate-methyl (meth)acrylate copolymers; and chlorine-containing synthetic resins, e.g., polyvinyl chloride, polyvinylidene chloride. The cylindrical tube can further have internal projections, slits, holding means, or catching means for use in disposing and fixing a pest control material. The portion where the pest control material is disposed in the cylindrical tube may be formed as a removal cassette, or the cylindrical tube may be vertically separated into two parts, the upper part of which is openable, to facilitate the disposition of a pest control material.

In the method of the present invention, for the purpose of attaining stable and lasting excellent effects on the control of pests, the value of the expression:

$$a \times b / c$$

where $a$ is the surface area (m$^2$) of a support, $b$ is the velocity (m/sec.) of a relative air current on the surface of a pest control material, and $c$ is the amount (g) of an active agent carried on the support, is preferably set in the range of 0.001 to 10, more preferably 0.01 to 1.

The pests which can be controlled by the method of the present invention include Arthropods, for example, various harmful insects, harmful ticks and mites, such as sanitary harmful insects, e.g., flies, mosquitoes, cockroaches; wood harmful insects; and food harmful insects.

Examples

The present invention will be further illustrated by the following Example; however, the present invention is not limited to this Example.

Example 1

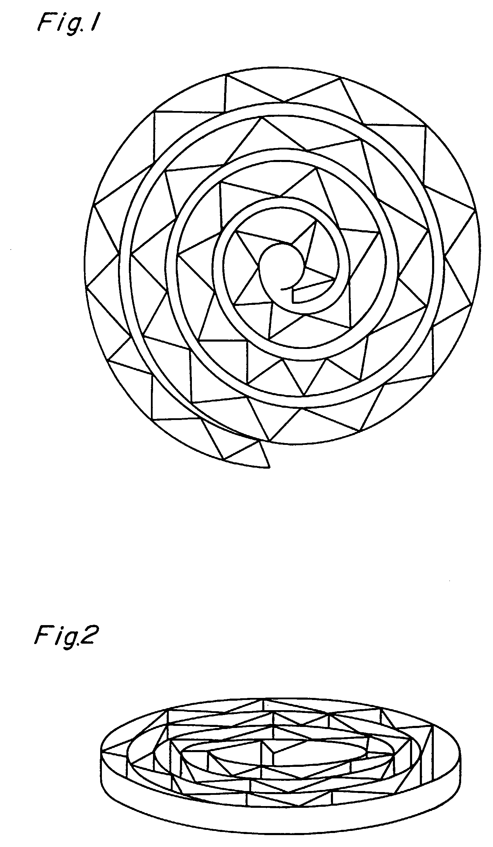

A spiral-shaped material having a diameter of 5.5 cm and a width of 0.5 cm was prepared by rolling up a piece of paper work with a honeycomb structure having a size of 0.5 cm x 69 cm x 0.5 cm and a surface area of 0.0138 m$^2$ from its one end. A solution of 150 mg of 1-ethynyl-2-methyl-2-pentenyl 3-(2-chloro-2-fluorovinyl)-(1R)-trans-2,2-dimethylcyclopropanecarboxylate (compound 1) in acetone was uniformly coated on the material, followed by air drying to remove the acetone. This gave pest control material 1 for test use having a surface area of 0.0138 m$^2$ as shown in Figs. 1 and 2.

In the same manner, pest control materials 2, 3, and 4 for test use were prepared, respectively, for 2,3,5,6-tetrafluoro-4-methylbenzyl (1R)-trans-3-(2-methyl-1-propenyl)-2,2-dimethylcyclopropanecarboxylate (compound 2), 2,3,5,6-tetrafluoro-4-methylbenzyl (1R)-trans-3-(2-chloro-2-fluorovinyl)-2,2-dimethylcyclopropanecarboxylate (compound 3), and 2,3,5,6-tetrafluoro-4-methylbenzyl (1R)-trans-3-((Z)-1-propenyl)-2,2-dimethylcyclopropanecarboxylate (compound 4). In addition, a pest control material for comparison was prepared in the same manner for 1-ethynyl-2-methyl-2-pentenyl (1R)-3-(2-methyl-1-propenyl)-2,2-dimethylcyclopropanecarboxylate (common name, empenthrin) as a comparative compound.

Each of the pest control materials thus prepared was disposed in the upper portion of a metal cylindrical tube having an inner diameter of 6.5 cm and a length of 8 cm with an electric fan provided in the lower portion thereof so that the spiral-patterned plane of each pest control material for test use is exposed to the wind blowing in the perpendicular direction from the electric fan positioned below. This was used as a test device.

The insecticidal test was conducted as follows: Ten female adults of common mosquitoes (*Culex pipens*

*pallens*) were set free in a glass tube having a diameter of 4 cm and a height of 12 cm, and both ends of the glass tube were closed with nylon nets. Two such glass tubes were prepared and placed in a plastic cylindrical cover having a diameter of 18 cm and a height of 30 cm. A metal cylindrical tube having a diameter of 20 cm and a height of 80 cm was disposed under the cylindrical cover and provided with the above test device at the bottom of the metal cylindrical tube. The fan in the device was driven for five minutes to allow a wind to blow at a velocity of 1.5 m/sec. The number of common mosquitoes knocked down after five minutes was determined, and the percent knockdown was calculated. The results are shown in Table 1.

TABLE 1

| Pest control material | Percent knockdown |
| --- | --- |
| 1 | 100% |
| 2 | 80% |
| 3 | 95% |
| 4 | 90% |
| for comparison | 5% |

As can be seen from Table 1, pest control materials 1, 2, 3, and 4 exhibited excellent effects on the control of common mosquitoes, while the pest control material for comparison exhibited very poor effects. This clearly demonstrates that according to the method of the present invention, it becomes possible to attain excellent effects on the control of pests without using any heat source.

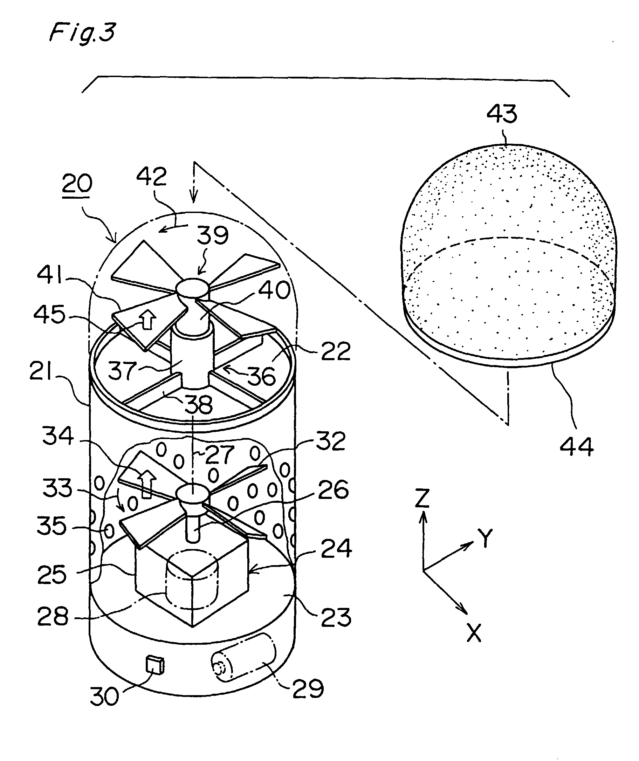

Next, with reference to the drawings, pest control apparatuses according to the present invention will be described hereinafter. Referring to Fig. 3, there is shown a pest control device of the first embodiment according to the present invention, generally indicated by reference numeral 20. The pest control device 20 has a cylindrical vertical housing 21. The housing 21 is opened at its top opening 22 but closed at its bottom with a partition 23 fixed in the housing 21. The partition 23 supports a drive mechanism generally indicated at 24. The drive mechanism 24 includes a case 25 fixed on the partition 23. The case 25 supports a vertical shaft 26 for rotation about its longitudinal axis 27. Also, the case 25 includes therein a motor 28 drivingly connected with the vertical shaft 26 so that, when the motor 28 is energized, the vertical shaft 26 rotates about its longitudinal axis 27. Preferably, the motor 28 is connected with the shaft 26 through a suitable reduction mechanism such as well-known pulley and belt mechanism or gear mechanism. The motor 28 is electrically connected with a power source such as battery 29. In this embodiment, although the battery 29 is removably provided below the partition 23, it may be mounted on the partition 23. In any event, the battery 29 should be positioned as it can be exchanged with a new one without any difficulty. The motor 28 and the battery 29 should be connected through a manual switch 30 preferably provided in the vertical housing 21 so that, when the switch 30 is turned on, the battery 29 supplies a predetermined voltage to the motor 28. Instead of battery 29, a commercial power may be available.

The vertical shaft 26 bears a plurality of blades 32 or propellers extending outwardly and radially at equal angles from a top portion of the vertical shaft 26. In this case, although four blades 32 are provided, the present invention is not limited thereto. Each blade 32 is inclined to an imaginary plane, i.e., X-Y plane in Fig. 3, perpendicular to the vertical shaft 26 extending in the vertical direction indicated at Z. This allows that, when the vertical shaft 26 as well as blades 32 rotates in a direction indicated at 33, the blade 32 generates a stream of air indicated by an arrow at 34 toward the top opening 22 of the housing 21. A plurality of cut-outs or air inlets 35 should be formed in the housing 21 so that a sufficient air can be drawn from the exterior into the interior of the housing 21. Preferably, the air-inlets 35 are formed below the blades 32. Another air-inlets may also be formed in the partition 23. In this instance, additional air-inlets should be made in a portion of the housing 21 located under the partition 23.

A bearing member generally indicated at 36 is provided at the top opening 22 of the housing 21. The bearing member 36 includes a bearing cylinder 37 extending vertically. The bearing cylinder 37 is fixedly supported at the center of the opening 22 in the housing 21 using a plurality of bridges 38 each extending between an inner surface of the housing 21 and an outer surface of the bearing cylinder 37. Although four bridges are provided at equal angles, the number of which is not restrictive provided that the bearing cylinder 37 can be supported positively.

The bearing cylinder 37 supports a pest agent carrier generally indicated at 39. The carrier 39 is entirely or partially made of the support material including the above-described pest control active agent. The carrier 39 has a shaft 40. The shaft 40 is sized to have an outer diameter slightly smaller than an inner diameter of the bearing cylinder 37, which allows the carrier 39 to be removed therefrom without any difficulty and the shaft 40 to rotate freely in the bearing cylinder 37. To help the shaft 40 rotate smoothly, i.e., to minimize a frictional force generated between opposing surfaces of the shaft 40 and the bearing cylinder 37, the inner surface of the bearing cylinder 37 may be coated with a low-frictional material such as polytetrafluoroethylene.

The shaft 40 supports a plurality of blades 41 or propellers extending outwardly and radially at equal angles from a top portion of the shaft 40. Although four blades 41 are provided to the shaft 40, the present invention is not limited thereto. Each blade 41 is inclined to the horizontal plane, i.e., X-Y plane in Fig. 3, perpendicular to the vertical shaft 40. This allows that, when the blades 41 catch the air stream 34, they provide the carrier 39 with a rotational force in a direction indicated at 42. This in turn allows the carrier 39 to make a dynamic contact with air that helps the pest active agent to vaporize and then diffuse into the atmosphere.

For safe, the carrier 39 is suitably protected by a breathable net or mesh 43. Preferably, the mesh 43 is configured in the form of dome leaving a space between the mesh 43 and the rotatable blades 41 so that it does not prevent the rotation of the carrier 39. Also, preferably, the domed mesh 43 is provided at its bottom peripheral edge with a ring 44 made of elastic metal or resin so that it can easily be fitted at the top portion of the housing 21. It is to be noted that a mesh size of the mesh 43 should be determined so as not to prevent the vaporized pest agent from diffusing into the atmosphere.

In operation, when the switch 30 is turned on, the motor 28 is energized to rotate the vertical shaft 26 and the blades 32 in the direction at 33, which generates the upward air stream 34 in the housing 21. The generated air stream 34 brings into contact with the surface of the blades 41 of the carrier 39, which forces the blades 41 and the carrier 39 to rotate in the direction of 42. The air steam 34 helps the pest control active agent vaporize. In addition, the rotation of the carrier 39, in particular blades 41, brings about a stream of air 45 relative to the surface of the carrier 39, which accelerates the vaporization of the pest control active agent in the earner.

The vaporized pest control agent is diffused by the air streams 34 and 45 into the atmosphere through the protection mesh 43. If all of or substantial part of the pest control active agent in the carrier has been vaporized, the mesh 43 is removed and then a new carrier 39 is replaced by the old one.

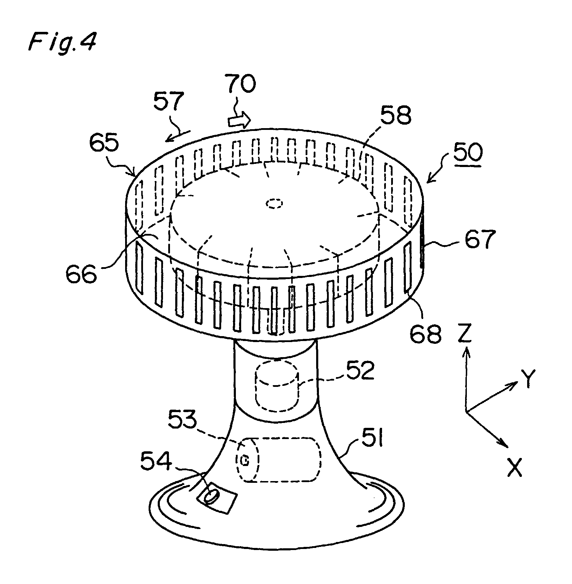

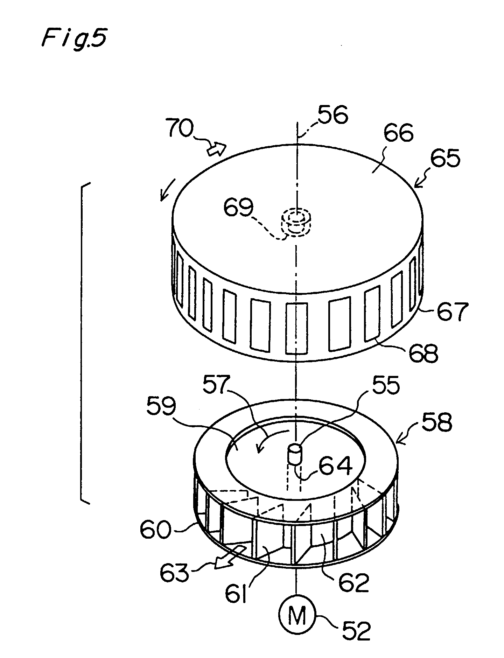

Referring to Figs. 4 and 5, there is shown a second embodiment of the pest control device according to the present invention, generally indicated by reference numeral 50. The pest control device 50 has a housing 51 in the form of truncated cone. The housing 51 includes a motor 52 fixed therein and a battery 53 removably mounted therein. The motor 52 and the battery 53 are electrically connected through a manual switch 54 provided in the housing 51 so that, when the switch 54 is turned on, the motor 52 is supplied with a predetermined voltage from the battery 53. Similar to the first embodiment, the housing 51 should be designed and constructed so that the battery 53 can be removed and mounted without any difficulty. Also, it is to be understood that the commercial power supply may be utilized instead of the battery 53.

As best shown in Fig. 5, the housing 51 has a vertical shaft 55 extending along a vertical axis 56 and supported for rotation about the vertical axis 56. The vertical shaft 55 is drivingly connected with the motor 52 so that, when the motor 52 is energized, the vertical shaft 55 rotates about the vertical axis 56 in the direction indicated by an arrow 57. As described in the previous embodiment, a suitable reduction mechanism such as pulley and belt may be provided between the motor 52 and the vertical shaft 55.

The vertical shaft 55 securely holds a centrifugal fan generally indicated by reference numeral 58. As best shown in Fig. 5, the centrifugal fan 58 has a top circular plate 59 and a bottom ring plate 60 spaced a predetermined distance from the top circular plate 59. Preferably, the top circular plate 59 and the ring plate 60 have substantially the same outer diameter. Positioned between the top circular plate 59 and the bottom ring plate 60 are a number of fins 61 securely connecting between top and bottom plates, 59 and 60. Each of the fins 61 is oriented obliquely against a radial direction of the circular plate 59 so that, when the centrifugal fan 58 rotates about an axis extending vertically at the center of the circular plate 59, it draws air in an inner chamber 62 surrounded by fins 61 and then blows it outwardly and radially. The blown air is indicated in Fig. 5 by an arrow 63. In addition, the top circular plate 59 has a through-hole 64 formed at the center of the plate. The through-hole 64 securely receives the vertical shaft 55, which allows the fan 58 to rotate based upon the rotation of the vertical shaft 55. The vertical shaft 55 is projected a predetermined length from the top circular plate 59.

The device 50 further includes a carrier generally indicated at 65. The carrier 65, which is entirely or partially made from the support material including the above-described pest control agent, has a circular plate 66 and a cylindrical vertical wall 67 or skirt extending downwardly from a peripheral edge of the circular plate 66. The circular plate 66 as well as the vertical wall 67 is sized to have an inner diameter that is greater than an outer diameter of the centrifugal fan 58 so that the carrier 65 surrounds and covers the centrifugal fan 58 when it takes an operational position shown in Fig. 4. The vertical wall 67 has a number of openings 68. Although each opening 68 is in the form of rectangular, it may be other configurations. The top circular plate 66 has a cylindrical bearing 69 at its center in a bottom surface thereof. The cylindrical bearing 69 has an inner diameter slightly greater than an outer diameter of the top projected portion of the vertical shaft 55. This allows the vertical shaft 55 to receive the cylindrical bearing 69 of the carrier 65, so that the carrier 65 can rotate about the vertical shaft 55. This also ensures that, when the vertical shaft 55 rotates, a frictional force generated between opposing surfaces of the vertical shaft 55 and the cylindrical bearing 69 allows the carrier 65 to rotate in the same direction around the vertical shaft 55.

In operation, when the switch 54 is turned on, the motor 52 is energized by the voltage supplied from the battery 53 to rotates the vertical shaft 55 in the direction of arrow 57. This results in the frictional force between the contact portions of the vertical shaft 55 and the cylindrical bearing 69, which in turn allows the carrier 65 to rotate in the same direction. At the rotation of the carrier 65, the surface of which makes a dynamic air-contact with air. Namely, an air stream 70 is generated relative to the surface of the carrier 65. The air stream 70 helps the pest control active agent vaporize and then diffuse into the air. The rotation of the vertical shaft 55 is also transmitted to the fan 58. The fan 58, when it rotates in the direction of arrow 57, draws air from the chamber 62 to blow it outwardly and obliquely toward the vertical wall 67 of the carrier. This accelerates the pest control active agent vaporize. The active agent so vaporized is then diffused into the atmosphere.

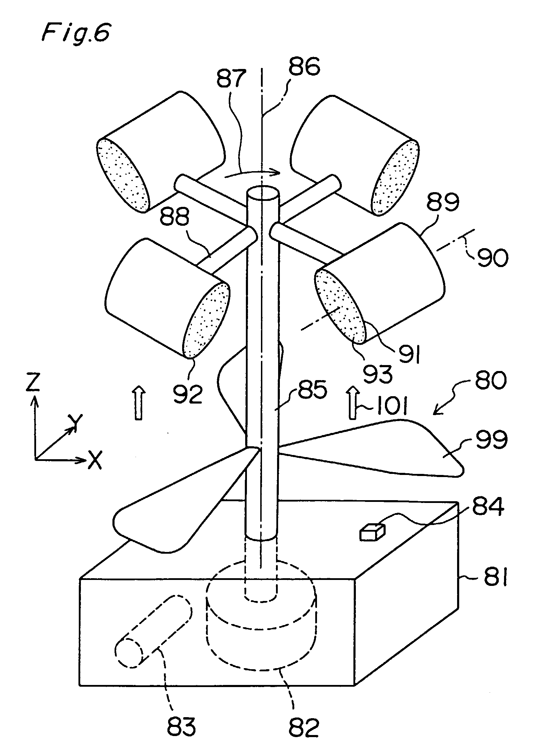

Referring to Fig. 6, there is shown a third embodiment of the pest control device according to the present invention, generally indicated by reference numeral 80. The device 80 has a base housing 81 in the form of rectangular box. The housing 81 includes a motor 82 fixed therein and a battery 83 removably mounted therein. The motor 82 and the battery 83 are electrically connected through a manual switch 84 provided in the housing 81 so that, when the switch 84 is turned on, the motor 82 is supplied with a predetermined voltage from the battery 83. Similar to the first embodiment, the housing 81 should be designed and constructed so that the battery 83 can be removed and mounted without any difficulty. Also, it is to be understood that the commercial power supply may be utilized instead of the battery.

The housing 81 holds a vertical shaft 85 extending along a vertical axis 86 and supported for rotation about the vertical axis 86. The vertical shaft 85 is drivingly connected with the motor 82 so that, when the motor 82 is energized, the vertical shaft 85 rotates about the vertical axis 86 in the direction indicated by an arrow 87. As described in the previous embodiment, a suitable reduction mechanism such as pulley and belt may be provided between the motor 82 and the vertical shaft 85.

The vertical shaft 85 supports a plurality of arms 88 extending outwardly and radially from the vertical shaft 85 at regular angles. Although four arms 88 are provided in this embodiment, the present invention is not limited thereto. Each of the arms 88 supports a cylinder 89 having openings 91 and 92 at its opposite ends so that a longitudinal axis 90 of the cylinder 89 is oriented horizontally. A carrier 93, which is entirely or partially made from the support material including the above-described pest control agent, is positioned within each cylinder 89.

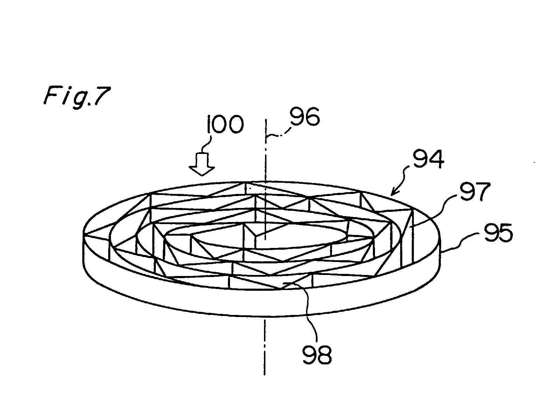

The carrier 93 includes one or more pest control active agent support members shown in Fig. 7. The support member, which includes the above-described pest control agent and is generally indicated at 94, is constructed by a plurality of cylindrical members 95 having different diameters and arranged about an axis 96 in a coaxial fashion. Each cylindrical member 95 is securely connected with the neighboring cylindrical member by a connecting member 97 running in a zigzag fashion between the cylindrical members, which results in a number of openings 98 between cylindrical members 95 and connecting members 97. The carrier 93 is removably inserted in the cylinder 89 with the axis 96 oriented toward the openings 91 and 92 so that, when the cylinder 89 rotates in the direction of arrow 87, the carrier 93 makes a dynamic air-contact with air at the surfaces of the members 95 and 97 at the openings 98, i.e., a stream of air is generated relative to the surfaces of the members 95 and 97.

It is to be understood that, although the support member 94 is made of cylindrical members each having different sizes from others, it may be formed by a spiral member and the connecting member running in a zigzag fashion between neighboring portions of the spiral member. Alternatively, the support member may be a honeycomb structure.

Further, the vertical shaft 85 supports a plurality of blades 99 or propellers extending outwardly and radially from the vertical shaft 85 at regular angles. Although three blades 99 are provided in this embodiment, the present invention is not limited thereto. Each blade 99 is inclined to a horizontal plane, i.e., X-Y plane in Fig. 6, so that when the vertical shaft rotates in the direction of arrow 87, an air of stream 101 is generated toward the cylinder 89.

In operation, when the switch 84 is turned on, the motor 82 is energized by the voltage supplied from the battery 83 to rotates the vertical shaft 85 as well as the cylinders 89 in the direction of arrow 87. This results in that the pest control agent support member 94 in the cylinder 89 makes a dynamic contact with air. Specifically, a stream of air shown in Fig. 7 by an arrow indicated at 100 is generated relative to the surfaces of the members 95 and 97. The stream of air 100 helps the pest control active agent vaporize. Then, the vaporized active agent is diffused into the atmosphere. In addition, the blades 99 rotate with the vertical shaft 85 to generate another stream of air 101, which accelerates the diffusion of the vaporized pest control active agent into the atmosphere.

It should be noted that, although the blades 99 are provided in this embodiment, they may be eliminated from the device 80. Also in this instance, the pest control agent can effectively be vaporized by the air stream 100 relative to the surfaces of the members 95 and 97.

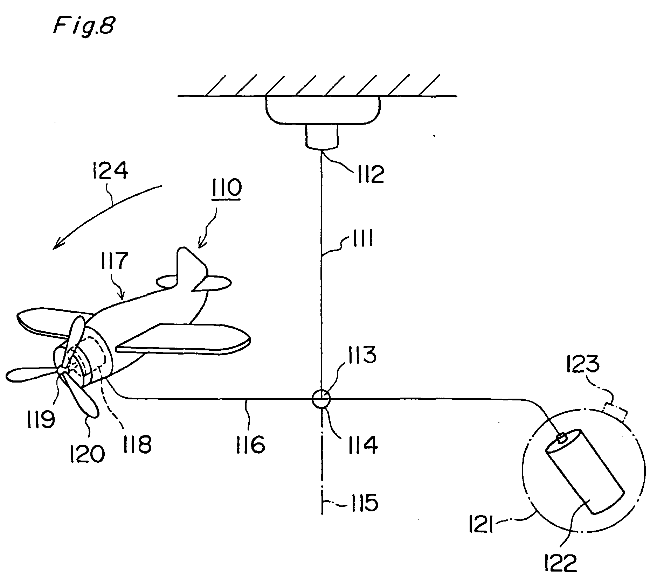

Referring to Fig. 8, there is shown a fourth embodiment of the pest control device according to the present invention, generally indicated by reference numeral 110. The device 110 includes a vertical shaft 111 having upper and lower ends, 112 and 113. The upper end 112 is securely connected to a suitable fixed portion such as ceiling. The lower end 113, on the other hand, has a coupling member 114 which is connected for rotation about a longitudinal axis 115 of the vertical shaft 111. The coupling member 114 holds a horizontal shaft 116 extending opposite directions. One end of the horizontal shaft 116 removably supports a carrier generally indicated at 117. The carrier 117, resembling an airplane in appearance for example, is entirely or partially made of the support material including the above-described pest control agent. The carrier 117 includes a motor 118 mounted therein. An output shaft 119 of the motor 118 supports a plurality of propellers 120. The other end of the horizontal shaft 116 carries a counterweight or battery holder 121 in which a battery 122 is removably mounted. The battery 122 is electrically connected to the motor 118 through an electric circuit positioned in the horizontal shaft 116. The circuit includes a manual switch 123 preferably provided in the battery holder 121.

In operation, when the switch 123 is turned on, a predetermined voltage is supplied from the battery 122 through the power line to the motor 118, which energizes the motor 118 to rotate the output shaft 119 and also the propellers 120. The rotation of the propellers 120 generates a propelling force, which allows the carrier 117 together with the battery holder 121 to rotate in a direction indicated by an arrow 124. This in turn results that the carrier 117 makes a dynamic contact with air or a stream of air is generated relative to the surface of the carrier 117, which helps the pest control active agent vaporize. The vaporized active agent is then diffused by the atmospheric air disturbed by the movements of the carrier 117 and battery holder 121.

In view of above, it will be seen that the several objects of the invention are achieved and other advantages results attained.

As various changes could be made in the above constructions without departing from the scope of the invention, it is intended that all matter contained in the above description or shown in the accompanying drawings shall be interpreted as illustrative and not in a limiting sense.

**Claims**

1.  A pest control method which comprises raising a relative air current on the surface of a pest control material carrying on a support at least one active agent selected from the group consisting of 1-ethynyl-2-methyl-2-pentenyl 3-(2-chloro-2-fluorovinyl)-2,2-dimethylcyclopropanecarboxylate, 2,3,5,6-tetrafluoro-4-methylbenzyl 3-(2-methyl-1-propenyl)-2,2-dimethylcyclopropanecarboxylate, 2,3,5,6-tetrafluoro-4-methylbenzyl 3-(2-chloro-2-fluorovinyl)-2,2-dimethylcyclopropanecarboxylate, and 2,3,5,6-tetrafluoro-4-methylbenzyl 3-(1-propenyl)-2,2-dimethylcyclopropanecarbox ylate, to vaporize and diffuse the active agent in the air.

2.  A pest control method according to claim 1, wherein the relative air current is raised by allowing a wind to blow the pest control material by the use of a blowing means.

3.  A pest control method according to claim 1 or 2, wherein the relative air current is raised by allowing the pest control material to move in the air.

4.  A pest control method according to claim 2 or 3, wherein the pest control material is disposed in a cylindrical tube having both open ends and the blowing means is provided at one of the open ends of the cylindrical tube.

5.  A pest control method according to any one of the preceding claims, wherein the expression:

$$a \times b / c$$

where $a$ is the surface area (m$^2$) of the support, $b$ is the velocity (m/sec.) of the relative air current on the surface of the pest control material, and $c$ is the amount (g) of the active agent carried on the support, takes a value in the range of 0.001 to 10.

6.  A pest control method according to any one of the preceding claims, wherein the support is made of paper, nonwoven fabric, cloth, or a resin film.

7.  A pest control method, comprising the steps of:

    providing a carrier having a pest control agent capable of vaporizing by the contact with air; and
    moving said carrier and thereby generating a stream of air relative to said carrier.

8.  A pest control apparatus, comprising:

    a carrier having a pest control agent capable of vaporizing by the contact with air; and
    means for moving said carrier and thereby generating a stream of air relative to said carrier.

9.  A pest control apparatus, comprising:

    a carrier including a pest control agent capable of vaporizing by the contact with air, said carrier comprising a shaft and a portion, said portion being securely supported by said shaft and extending substantially outwardly and radially from said shaft; and
    motor for rotating said carrier about said shaft, which generates a stream of air relative to said carrier to help said pest control agent vaporize.

10. A pest control apparatus, comprising:

    a carrier including a pest control agent capable of vaporizing by the contact with air, said carrier comprising a shaft and a blade, said blade being securely supported by said shaft and extending substantially outwardly and radially from said shaft, said blade being inclined to a plane perpendicular to said shaft; a bearing for supporting said carrier so that said carrier can rotate about said shaft; a motor; and a fan drivingly connected with said motor so that said fan rotates to provide said inclined blade of said carrier with a stream of air which causes said carrier to rotate and thereby generates another stream of air relative to said carrier to help said pest control agent vaporize.

11. A pest control apparatus according to claim 10, wherein said carrier includes a plurality of said blades equally positioned about said shaft, so that said carrier is in the form of propeller.

12. A pest control apparatus according to claim 10 or 11, further comprises a breathable member surrounding said carrier.

13. A pest control apparatus, comprising:

a carrier including a pest control agent capable of vaporizing by the contact with air, said carrier comprising a bearing portion, a horizontal portion extending substantially outwardly and radially from said bearing portion, and a vertical portion extending substantially downwadly from said horizontal portion; a motor; a shaft drivingly connected with said motor for rotation and being engaged with said bearing portion so that said carrier can rotate about said shaft by a frictional contact between said bearing portion and said shaft generated at the rotation of said shaft, thereby forming a stream of air relative to said carrier to help said pest control agent vaporize; and a fan securely supported by said shaft so that said fan rotates together with said shaft to provide said carrier with an additional stream of air which accelerates a vaporization of said carrier.

14. A pest control apparatus according to claim 13 wherein said fan is a centrifugal fan and positioned inside said carrier.

15. A pest control apparatus, comprising:

a rotatable shaft; an arm securely supported by said shaft and extending outwardly and radially from said shaft; a carrier supported by said arm and including a pest control agent capable of vaporizing by the contact with air; and a motor for rotating said shaft, which gives rise to a stream of air relative to said carrier to help said pest control agent vaporize.

16. A pest control apparatus according to claim 15, wherein said shaft securely supports a blade inclined to a plane perpendicular to said shaft, which gives rise to an additional stream of air to accelerates a vaporization of said carrier

17. A pest control apparatus, comprising:

a vertical member having first and second ends, said vertical member being fixed at said first end; a horizontal member connected to said second end of said vertical member so that said horizontal member can rotate about said second end of said vertical member, said horizontal member having first and second arms extending opposite directions from said second end of said vertical member; a carrier supported by said first arm of said horizontal member and including a pest control agent capable of vaporizing by the contact with air; a counterweight supported by said second arm of said horizontal member so that said counterweight is balanced against said carrier; means for rotating said horizontal member about said second end of said vertical member and thereby generating a stream of air relative to said carrier.

18. A pest control apparatus according to claim 17, wherein said means for rotating said horizontal member includes a motor mounted in said carrier and a propeller drivingly connected with said motor, said counterweight includes a battery for providing electricity to said motor, and an electric circuit connecting between said motor and said battery.

*Fig. 1*

*Fig.2*

*Fig.3*

*Fig.4*

## Fig.5

Fig.6

*Fig.7*

Fig.8

## EUROPEAN SEARCH REPORT

**European Patent Office**

**Application Number**

EP 99 11 0212

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | EP 0 344 768 A (SUMITOMO CHEMICAL CO) 6 December 1989 (1989-12-06) * page 2, line 6 - line 31 * * page 4, line 31 - line 35 * * page 6; table 1 * --- | 1-6 | A01N53/00 A01N25/18 A01M1/20 A61L9/12 //(A01N53/00, 25:18) |
| Y | EP 0 378 026 A (ROUSSEL UCLAF) 18 July 1990 (1990-07-18) * page 2, line 4 - line 35 * * page 4, line 39 - line 40 * * page 6; example 3 * --- | 1-6 | |
| Y | DE 39 38 664 A (SUMITOMO CHEMICAL CO) 31 May 1990 (1990-05-31) * page 2, line 25 - line 39 * * page 3, line 50 - line 60 * --- | 1-6 | |
| Y | EP 0 775 441 A (EARTH CHEMICAL CO) 28 May 1997 (1997-05-28) * page 3, line 20 - line 30 * * page 6, line 44 - line 46 * * page 6, line 57 - line 58 * | 1-6 | |
| D | & WO 96 04786 A --- | | **TECHNICAL FIELDS SEARCHED (Int.Cl.6)** A01N A01M |
| Y | WO 96 32843 A (JOHNSON & SON INC S C) 24 October 1996 (1996-10-24) * page 9, paragraph 2 * * page 11, paragraph 3 * --- | 1-4 | A61L |
| Y | EP 0 792 581 A (SUMITOMO CHEMICAL CO) 3 September 1997 (1997-09-03) * page 2, line 26 - line 32 * * page 2, line 39 - line 40 * --- | 1,2,4 | |
| X | US 5 480 591 A (LAGNEAUX PATRICK ET AL) 2 January 1996 (1996-01-02) * column 1, line 7 * * column 2, line 5 - column 3, line 38 * --- | 7-9,15, 16 | |

-/--

The present search report has been drawn up for all claims

| Place of search | Date of completion of the search | Examiner |
|---|---|---|
| THE HAGUE | 21 September 1999 | Lamers, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 99 11 0212

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE 297 12 821 U (NENTWIG WALTER) 30 October 1997 (1997-10-30) * page 1, line 1,2 * * page 3, line 1 - line 10 * * page 4, line 17 - line 20 * | 7-9,15, 16 | |
| Y | --- | 10-14 | |
| Y | NL 47 732 C (E.GEHRCKE) 16 February 1940 (1940-02-16) * figures 3-5 * ----- | 10-14 | |
| | | | **TECHNICAL FIELDS SEARCHED** (Int.Cl.6) |

The present search report has been drawn up for all claims

| Place of search | Date of completion of the search | Examiner |
|---|---|---|
| THE HAGUE | 21 September 1999 | Lamers, W |

EPO FORM 1503 03.82 (P04C01)

EP 0 962 139 A1

## ANNEX TO THE EUROPEAN SEARCH REPORT
## ON EUROPEAN PATENT APPLICATION NO.

EP 99 11 0212

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-1999

| Patent document cited in search report | | Publication date | Patent family member(s) | | Publication date |
|---|---|---|---|---|---|
| EP 0344768 | A | 06-12-1989 | JP | 2022248 A | 25-01-1990 |
| | | | JP | 2546339 B | 23-10-1996 |
| | | | US | 4985457 A | 15-01-1991 |
| EP 0378026 | A | 18-07-1990 | FR | 2641275 A | 06-07-1990 |
| | | | AU | 4707189 A | 05-07-1990 |
| | | | CA | 2006835 A | 29-06-1990 |
| | | | CN | 1044936 A | 29-08-1990 |
| | | | JP | 2225442 A | 07-09-1990 |
| | | | MX | 18761 A | 01-11-1993 |
| DE 3938664 | A | 31-05-1990 | AU | 627792 B | 03-09-1992 |
| | | | AU | 4478789 A | 31-05-1990 |
| | | | CH | 677717 A | 28-06-1991 |
| | | | FR | 2639187 A | 25-05-1990 |
| | | | GB | 2225533 A | 06-06-1990 |
| | | | GR | 89100767 A | 31-12-1990 |
| | | | JP | 2282309 A | 19-11-1990 |
| EP 0775441 | A | 28-05-1997 | AU | 3191695 A | 07-03-1996 |
| | | | BR | 9508867 A | 06-01-1998 |
| | | | CA | 2197191 A | 22-02-1996 |
| | | | CN | 1159736 A | 17-09-1997 |
| | | | HU | 77789 A | 28-08-1998 |
| | | | WO | 9604786 A | 22-02-1996 |
| WO 9632843 | A | 24-10-1996 | AU | 5442396 A | 07-11-1996 |
| | | | BR | 9608105 A | 09-02-1999 |
| | | | CA | 2217966 A | 24-10-1996 |
| | | | CN | 1183709 A | 03-06-1998 |
| | | | EP | 0824318 A | 25-02-1998 |
| | | | HU | 9900059 A | 28-05-1999 |
| | | | JP | 11504627 T | 27-04-1999 |
| | | | NZ | 306219 A | 26-06-1998 |
| | | | PL | 322743 A | 16-02-1998 |
| EP 0792581 | A | 03-09-1997 | AU | 1488397 A | 04-09-1997 |
| | | | BR | 9701091 A | 15-12-1998 |
| | | | CN | 1166272 A | 03-12-1997 |
| | | | JP | 9289855 A | 11-11-1997 |
| | | | JP | 9308421 A | 02-12-1997 |
| US 5480591 | A | 02-01-1996 | FR | 2717392 A | 22-09-1995 |
| | | | AT | 181243 T | 15-07-1999 |
| | | | AU | 668494 B | 02-05-1996 |
| | | | AU | 1486095 A | 12-10-1995 |

EPO FORM P0459

For more details about this annex : see Official Journal of the European Patent Office, No. 12/82

19

**ANNEX TO THE EUROPEAN SEARCH REPORT
ON EUROPEAN PATENT APPLICATION NO.**

EP 99 11 0212

This annex lists the patent family members relating to the patent documents cited in the above–mentioned European search report.
The members are as contained in the European Patent Office EDP file on
The European Patent Office is in no way liable for these particulars which are merely given for the purpose of information.

21-09-1999

| Patent document cited in search report | | Publication date | Patent family member(s) | | Publication date |
|---|---|---|---|---|---|
| US 5480591 | A | | BR 9507089 A | | 09-09-1997 |
| | | | CA 2144697 A,C | | 17-09-1995 |
| | | | CN 1143912 A | | 26-02-1997 |
| | | | DE 69510253 D | | 22-07-1999 |
| | | | EP 0672425 A | | 20-09-1995 |
| | | | WO 9524935 A | | 21-09-1995 |
| | | | JP 9512443 T | | 16-12-1997 |
| DE 29712821 | U | 30-10-1997 | NONE | | |
| NL 47732 | C | | DE 634697 C | | |
| | | | GB 457329 A | | |

EPO FORM P0459

For more details about this annex : see Official Journal of the European Patent Office, No. 12/82

20